# EUROPEAN PATENT APPLICATION

(11) **EP 4 685 705 A1**
(43) Date of publication of application: **28.01.2026**
(21) Application number: 25188143.9
(22) Date of filing: 08.07.2025
(51) Int. Cl.: G06N 3/084

(54) **SYSTEM FOR INTRODUCING A TARGET PERSON TO MODIFY ACTIONS AND METHOD OF DOING THE SAME**

(30) Priority: 24.07.2024 JP 2024118381
(71) Applicant: Cognitive Research Labs, Inc., Tokyo 1060032 (JP)
(72) Inventor: TOMABECHI, Hideto, Tokyo, 1060032 (JP)
(74) Representative: Winter, Brandl - Partnerschaft mbB

(57) **Abstract**

A system (300) for designing intervention(s) to introduce a target person (172) to modify his/her actions, includes a first device (132) for storing biometric information about the target person (172), the biometric information including reactions having been made by the target person (172) in response to past external stimulation directed to the target person (172), a second device (131A) for recreating thought/action pattern of the target person (172) by means of prediction model (210) having been machine-learned using the biometric information as teacher data (200), a third device (131B) receiving present external stimulation and/or expected future external simulation directed to the target person (172), and outputting expected reactions of the target person (172) to be made in response to those external stimulation, and a fourth device (131C) for designing intervention(s) to introduce the target person (172) to take target action(s) in dependence on the expected reactions.

## Description

### BACKGROUND OF THE INVENTION

### FIELD OF THE INVENTION

The present invention relates to a system for designing intervention(s) to introduce a target person to modify his/her actions to target (preferable) actions. Specifically, the system (a) machine-learns thought/action pattern(s) having been made by a target person, based on past words and actions having been made by the target person in response to external stimulation directed to the target person, (b) predicts words and actions of the target person when present external stimulation and/or future expected external stimulation are/is directed to the target person, and (c) when the words and actions are not preferable, designs intervention(s) to introduce the target person to modify his/her actions to target (preferable) actions.

The present invention relates further to a method of doing the same, and a computer program for causing a computer to carry out the method.

### DESCRIPTION OF THE RELATED ART

U.S. Department of Defense (Pentagon) has announced the document named "Psychological Operations" dated January 7, 2010.

In a category of a concept of "Psychological Operations" defined in the document, disinformation (fake information) to be spread by "State Player" sponsored by military and/or government is recognized to be main weapon or one of new tactical weapons in "Psychological Operations".

It is possible to cause a government and/or military of enemy to misjudge by spreading disinformation to thereby make it possible to assist physical attacks such as launching a missile to be made by home country. Thus, spreading disinformation is recognized as a new tactical weapon in place of physical attacks.

In the modern era having shifted from the age of "Psychological Operations" to the age of cognitive warfare, information (including disinformation) serves as a strategic weapon aimed at altering perceptions of each individual, including the leadership and citizens of adversary nation(s), to align with the goals of home country, and is used to attack individual cognition with a target of civilian populations of adversary nation(s), and further, a target of shaping global public opinion.

Psychological Operations primarily relates to the dissemination of disinformation as a tactical weapon within existing domains of warfare such as land, sea, and air. However, in modern cognitive warfare, cognitive domain itself has become a battlefield. Thus, it is necessary to understand that, in addition to tactical aspects of disrupting or dismantling enemy leadership, cognitive warfare functions also as a strategic weapon targeting cognitive transformation of individual civilians. This is the same as that cyber technology, which was once an offensive tool supporting physical tactical weapons in traditional warfare, has evolved to make cyber domain itself a battlefield.

In modern cognitive warfare, generated and utilized information goes beyond merely disrupting command chain of adversary nation(s), and is integrally produced based on medium- to long-term national interest. In many cases, large quantities of information generated by AI, such as generative AI, are intricately combined and disseminated.

Additionally, information used today is not limited to linguistic information or other symbolic information, but also includes quasi-symbolic information such as subliminal messages. Furthermore, through non-symbolic brain information processing, such as biofeedback, it is becoming possible to directly intervene in thoughts of a target person and rewrite his/her information.

To leverage or defend against such attacks, it is necessary to abstract patterns out of vast amounts of information of different degrees of abstraction, including sensory and linguistic data, recognize enemy's intentions, and further, make use of the information. To this end, it is necessary to operate a plurality of Als in a cooperative and distributed manner to thereby perform inference and analysis at all levels of abstraction.

As described above, in the modern era, information, whether it is true or false, is a strategic weapon aimed at transforming cognition of individual citizens, including upper echelons of adversary nation(s), to align with goals of our country.

Moreover, not only in a field of strategic weaponry but also in a field of business, it is an extremely important strategy to handle information to align with objective.

The same can be said for context of individuals' everyday lives.

However, in both strategic and business domains, information aligning with such strategies is not yet fully utilized.

### SUMMARY OF THE INVENTION

In view of the above-mentioned present situation, it is an exemplary object of the present invention to provide both a system for utilizing information so as to align with such strategies as mentioned above, and a method of doing the same.

In a first exemplary aspect of the present invention, there is provided a system for designing intervention(s) to introduce a target person to modify his/her actions, including a first device for storing therein biometric information about the target person, the biometric information including reactions having been made by the target person in response to past external stimulation directed to the target person, a second device for recreating thought pattern and/or action pattern of the target person through the use of prediction model having been machine-learned using the biometric information as teacher data, a third device receiving, as input, present external stimulation and/or expected future external simulation directed to the target person, and outputting expected reactions of the target person to be made in response to those external stimulation, based on the thought pattern and/or action pattern having been recreated by the second device, and a fourth device for designing intervention(s) to introduce the target person to take target action(s) in dependence on the expected reactions of the target person having been output by the third device.

It is preferable that the biometric information includes brain pattern information of the target person.

It is preferable the fourth device designs at least one of first intervention and second intervention, the first intervention introducing the target person to weaken his/her emotion arisen by the external stimulation, the second intervention introducing the target person to replace his/her emotion arisen by the external stimulation with other emotion.

In a second exemplary aspect of the present invention, there is provided a portable wireless communication device including the above-mentioned system.

In a third exemplary aspect of the present invention, there is provided a promotion system for designing and carrying out intervention(s) to introduce a target person to modify his/her actions, including an introduction system and a portable wireless communication device owned by the target person, the introduction system including a first device for storing therein biometric information about the target person, the biometric information including reactions having been made by the target person in response to past external stimulation directed to the target person, a second device for recreating thought pattern and/or action pattern of the target person through the use of prediction model having been machine-learned using the biometric information as teacher data, a third device receiving, as input, present external stimulation and/or expected future external simulation directed to the target person, and outputting expected reactions of the target person to be made in response to those external stimulation, based on the thought pattern and/or action pattern having been recreated by the second device, and a fourth device for designing a policy of intervention(s) to introduce the target person to take target action(s) in dependence on the expected reactions of the target person having been output by the third device, the portable wireless communication device storing therein an application for designing intervention(s) based on the policy, the introduction system transmitting the policy to the portable wireless communication device of the target person, the application, on receipt of the policy, designing action of intervention(s) customized for the target person in accordance with the policy.

In a fourth exemplary aspect of the present invention, there is provided a method of designing intervention(s) to introduce a target person to modify his/her actions, including storing biometric information about the target person, the biometric information including reactions having been made by the target person in response to past external stimulation directed to the target person, recreating thought pattern and/or action pattern of the target person through the use of prediction model having been machine-learned using the biometric information as teacher data, receiving, as input, present external stimulation and/or expected future external simulation directed to the target person to thereby output expected reactions of the target person to be made in response to those external stimulation, based on the thought pattern and/or action pattern having been recreated in the recreating step, and designing intervention(s) to introduce the target person to take target action(s) in dependence on the expected reactions of the target person having been output in the receiving step.

It is preferable that at least one of first intervention and second intervention is designed in the designing step, the first intervention introducing the target person to weaken his/her emotion arisen by the external stimulation, the second intervention introducing the target person to replace his/her emotion arisen by the external stimulation with other emotion.

In a fifth exemplary aspect of the present invention, there is provided a method of designing intervention(s) to introduce a target person to modify his/her actions, including storing biometric information about the target person, the biometric information including reactions having been made by the target person in response to past external stimulation directed to the target person, recreating thought pattern and/or action pattern of the target person through the use of prediction model having been machine-learned using the biometric information as teacher data, receiving, as input, present external stimulation and/or expected future external simulation directed to the target person to thereby output expected reactions of the target person to be made in response to those external stimulation, based on the thought pattern and/or action pattern having been recreated in the recreating step, designing a policy of intervention(s) to introduce the target person to take target action(s) in dependence on the expected reactions of the target person having been output in the receiving step, and designing action of intervention(s) customized for the target person in accordance with the policy having been designed in the previous step, the action of intervention(s) being designed by an application stored in a portable wireless communication device owned by the target person.

In a sixth exemplary aspect of the present invention, there is provided a recording medium readable by a computer, storing a program therein for causing a computer to carry out the above-mentioned method.

As having been explained so far, the present invention has an object to modify action of a target person to a target action.

For example, in an example in which the present invention is applied to a military field, a user of the present invention can make enemy leaders perceive that a user's country holds an advantage, thereby leading the enemy leaders to decide to avoid war against the user's country. Alternatively, the user of the present invention could cause enemy leaders to unconsciously choose actions that favor the user's country, resulting in a sense of failure that their strategy has backfired.

A field to which the present invention can be applied is not limited to a military field, but may be a field of business. For instance, the present invention may be implemented in a field of marketing, enabling to encourage consumers to purchase our products.

In existing behavior modification approaches, that is, methods used to alter others' behavior to induce desired actions, particularly in behaviorist techniques, a predominant model has been a "event-driven type model," which predicts future events based on past occurrences. In this event-driven type model, since a process inferring output event having correlation with specific input event is used, time-series information of events is essential, leading to the following fundamental limitations.

The event-driven type model makes a response solely based on input events. In other words, the event-driven type model is inherently reactive. Consequently, it can only address known patterns or predictable events, preventing it from taking proactive measures.

For example, in military operations, this reactive stance results in a disadvantage, as it inevitably places us in a defensive position against enemy nations.

The event-driven type model relies exclusively on past data. As a result, it lacks flexibility to respond appropriately to unknown or unforeseen events.

For instance, in dealing with unprecedented events such as a novel virus pandemic or emergence of unknown technologies, past data alone is insufficient to devise countermeasures against them. This issue is particularly evident in fields of military and security, where ability to respond immediately to unpredictable new threats is required.

Moreover, in traditional behaviorist approaches, since human behavioral tendencies are abstracted to some extent and treated as a whole, it was impossible to address each of behavioral tendencies of individuals.

In inference systems such as large-scale language model (LLMs), specific individuals are not targeted, but a wide range of text, audio, image, and video data are used as input. These data are often creative works or descriptions expressing emotions, and they do not necessarily reflect actual human thought patterns accurately. Therefore, inference systems built on these general data do not always accurately represent thought or behavior patterns of specific individuals.

To address these issues, the present invention adopts an approach based on direct analysis of biometric information of a target individual (such as brain patterns obtained through brain-imaging devices), without relying on time-series event data.

In this approach, as described later, three types of inference systems having different roles (Thought Reproduction AI, Behavior Prediction AI, and Behavior Modification AI) are used. Each system analyzes individual biometric information (such as brain patterns) of a target person, predicts future behavior of a target person, and designs specific intervention measures for those behaviors.

Furthermore, in the present invention, as described later, a plurality of same-type inference systems are operated in parallel to more accurately reproduce a cognitive model of a target person. Through this parallelization, it is possible to reproduce a plurality of thought patterns inherent to a target person, and perform optimal inference according to the situation. Additionally, a likelihood of behavior modification in a target person can be predicted in real-time, allowing for rapid design of effective intervention measures.

In addition, by using a simulation-based behavior prediction system, the intervention measures proposed by the above-mentioned inference systems can be pre-simulated. By repeating validation and evaluation, it is possible to develop the most effective intervention measures.

This system demonstrates particular value in military domains where rapid decision-making is required and where a battlefield situation can change instantly. This enables the prediction of enemy attack intentions in advance and allows for the swift planning and execution of defensive actions.

As described above, the present invention is based on an approach in which biometric information of a target person is directly analyzed. Specifically, by moving away from conventional constraints that rely on time-series event data, and adopting a cognitive model architecture grounded in cognitive science, the present invention achieves the objective of determining "who, when, and how to prompt."

### ADVANTAGES OBTAINED BY THE INVENTION

In accordance with the present invention, biometric information of a target person such as brain patterns can be analyzed in real-time, making it possible to predict target's behavior before events occur, and guide the target person to modify his/her actions. This allows for formulation of strategic countermeasures ahead of a target person, ensuring that conventional reactive, belated responses can be overcoming.

In accordance with the present invention, since behavior prediction is directly performed based on current biometric information rather than relying on past event patterns, it is possible to quickly adapt to new events and changes to thereby provide timely countermeasures.

Additionally, because detailed biometric information of each of target persons is directly analyzed, the individual behavior of each target person can be predicted. This allows for more accurate understanding of the actual cognitive patterns and behavioral tendencies of each target person, enabling the creation of more precisely tailored intervention measures for each target person.

In accordance with the present invention, it is possible to generate customized interventions for each target person, thereby enhancing effectiveness of interventions for each target person.

In addition, during a process leading to intervention actions, workload can be distributed between cloud computers and edge computers, resulting in that use of computing resources can be reduced. This allows for efficient execution of large-scale campaigns or information dissemination activities with limited resources.

### BRIEF DESCRIPTION OF THE DRAWINGS

FIG. 1 is a block diagram illustrating an example of the system for introducing a target person to modify his/her actions to target (preferable) actions, in accordance with a first exemplary embodiment of the present invention.
FIG. 2 is a conceptual view showing a structure of the first program.
FIG. 3 is a conceptual view showing functions of the first to third programs.
FIG. 4 is a flowchart showing the operation of the system illustrated in FIG. 1.
FIG. 5 is a conceptual view showing the collaborative relation among the first to third programs.
FIG. 6 is a conceptual view showing construction and updating of a cognitive model constructed by the first program (thought reproduction AI).
FIG. 7 illustrates an example of biometric information of a target person.
FIG. 8 illustrates an example of behavioral information of a target person.
FIG. 9 illustrates an example of external stimulation directed to a target person.
FIG. 10 is a conceptual view of the behavior prediction system constructed by the second program.
FIG. 11 is a conceptual view of the behavior modification system constructed by the third program.
FIG. 12 is a conceptual view of the intervention delivery system.
FIG. 13 illustrates an example of input and output (a signal indicative of a policy of intervention, and actions of intervention) in a security area.
FIG. 14 illustrates an example of input and output (a signal indicative of a policy of intervention, and actions of intervention) in a marketing area.
FIG. 15 illustrates an example of input and output (a signal indicative of a policy of intervention, and actions of intervention) in an entertainment area.

### DESCRIPTION OF THE EXEMPLARY EMBODIMENTS

Exemplary embodiments in accordance with the present invention will be explained hereinbelow with reference to drawings.

### (First Exemplary Embodiment)

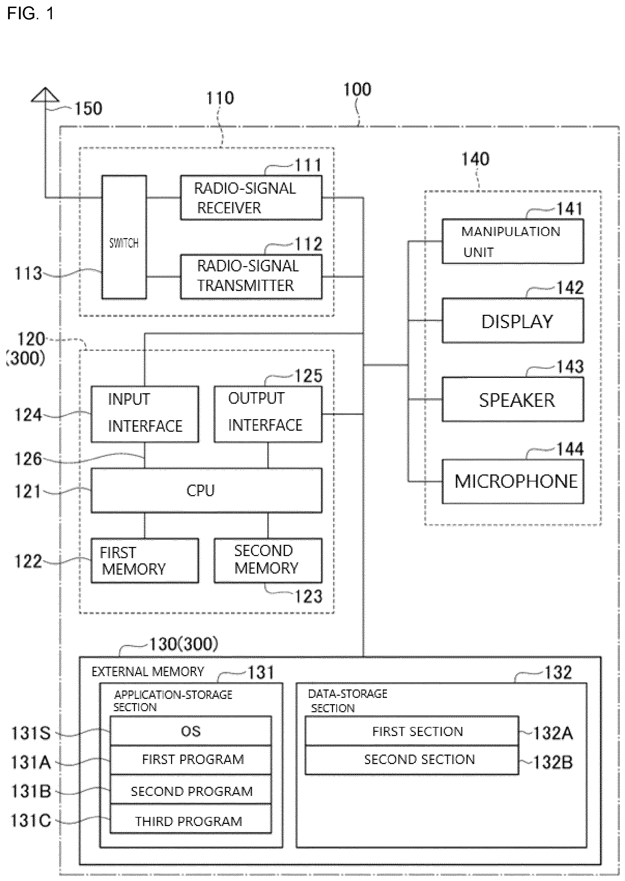

FIG. 1 is a block diagram illustrating an example of a structure of a portable wireless communication device 100 including therein a system 300 for designing intervention(s) to introduce a target person to modify his/her actions to target (preferable) actions, in accordance with the first exemplary embodiment of the present invention.

The portable wireless communication device 100 is designed to include a communication unit 110, a control unit 120, an external memory (a hard disc) 130, an input/output (IO) unit 140, and an antenna 150. The system 300 is constructed by the control unit 120 and the external memory (a hard disc) 130.

The portable wireless communication device 100 is configured, for instance, as a portable telephone device such as a cellular phone.

The communication unit 110 is connected to the antenna 150, and transmits data to and receives data from other wireless communication devices through the antenna 150 in radio-signal communication.

The communication unit 110 includes a radio-signal receiver 111, a radio-signal transmitter 112, and switch 113.

The radio-signal receiver 111 demodulates data received from other wireless communication devices, and then, transmits the demodulated data to the control unit 120. The radio-signal transmitter 112 modulates data output from the control unit 120, and then, transmits the modulated data to other wireless communication devices through the antenna 150. The switch 113 receives a control signal output from the control unit 120, and exchanges a transmission (modulation) mode to a receipt (demodulation) mode and vice versa in accordance with the received control signal.

The control unit 120 is comprised of a central processing unit (CPU) 121, a first memory 122 comprised of a read only memory (ROM), a second memory 123 comprised of a random access memory (RAM), an input interface 124 through which commands and/or data having been input into the control unit 120 are transmitted to the central processing unit 121, an output interface 125 through which results of computation having been executed by the central processing unit 121 are output, and buses 126 through which the central processing unit 121 is electrically connected with the first memory 122, the second memory 123, the input interface 124, and the output interface 125.

The first memory 122 stores therein both control programs to be executed by the central processing unit 121 and unrewritable data.

The second memory 123 stores therein various data and parameters, and presents a working area to the central processing unit 121. That is, data and/or programs temporarily necessary for the central processing unit 121 to execute control programs is(are) read out of the external memory 130, and temporarily stored in the second memory 123.

The central processing unit 121 entirely controls an operation of the portable wireless communication device 100 in cooperation with OS (Operating System). Specifically, the central processing unit 121 reads first to third programs 131A to 131C (detailed later) out of the external memory 130, and executes the first to third programs 131A to 131C. Thus, the central processing unit 121 works in accordance with the first to third programs 131A to 131C stored in the external memory 130. As mentioned later, the central processing unit 121 makes outputs in response to inputs by means of a trained evaluation model.

The IO unit 140 includes a manipulation device 141, a display 142, a speaker 143, and a microphone 144.

The manipulation unit 141 is comprised of a ten-key pad, for instance. Various data is input into the control unit 120 through the manipulation unit 141.

The display 142 is comprised of a liquid crystal display (LCD), for instance. The display 142 displays computation results carried out by the control unit 120, and various data.

Audio data having been received from other wireless communication devices is output through the speaker 143. Voice of a user of the portable wireless communication device 100 is collected through the microphone 144, and output to other wireless communication devices through the radio-signal transmitter 112.

The external memory (hard disc) 130 is comprised of an application-storage section 131 and a data-storage section 132.

The data-storage section 132 includes a first section 132A storing therein biometric information of a target person such as brain patterns, and a second section 132B storing therein various data about a target person other than the biometric information.

The application-storage section 131 stores therein OS (Operating System) 131S for controlling entire operation of the portable wireless communication device 100, a first program 131A, a second program 131B, and a third program 131C.

The first program 131A, the second program 131B and the third program 131C act as a thought reproduction AI, a behavior prediction AI, and a behavior modification AI, respectively, as mentioned later.

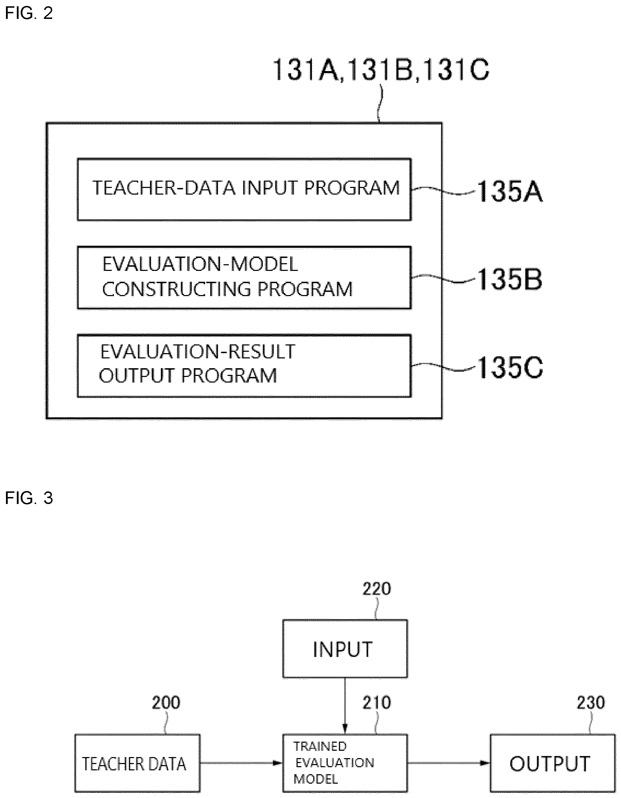

FIG. 2 is a conceptual view showing a structure of the first program 131A.

As illustrated in FIG. 2, the first program 131A is comprised of a teacher-data input program 135A, an evaluation-model constructing program 135B, and an evaluation-result output program 135C.

Similarly to the first program 131A, each of the second program 131B and the third program 131C is comprised of those three programs 135A, 135B and 135C.

The teacher-data input program 135A inputs teacher-data into the central processing unit 121 for constructing an evaluation model by machine learning.

The evaluation-model constructing program 135B uses teacher data having been input through the teacher-data input program 135A to thereby construct evaluation models by machine learning, and outputs the thus constructed trained evaluation models to the central processing unit 121.

For instance, neural network may be used as an algorithm for constructing an evaluation model.

The evaluation-result output program 135C outputs trained evaluation models having been constructed by the evaluation-model constructing program 135B, to the central processing unit 121.

FIG. 3 is a conceptual view showing functions of the first to third programs 131A to 131C.

As illustrated in FIG. 3, teacher data 200 is input into the evaluation-model constructing program 135B by the teacher-data input program 135A, and the evaluation-model constructing program 135B constructs an evaluation model 210 based on the teacher data 200.

The evaluation model 210 continuously carries out machine learning through the use of later-input teacher data 200, and turns into a trained evaluation model 210.

The trained evaluation model 210 receives data 220 in dependence on the first to third programs 131A, 131B and 131C. For instance, biometric information about a target person transmitted from the first section 132A and data about a target person other than the biometric information transmitted from the second section 132B are input as the data 220 into the first program 131A.

The trained evaluation model 210 makes evaluation results (for instance, thought patterns of a target person, behavior prediction of a target person, intervention action(s), and so on) based on the data 220, and outputs the evaluation results as output 230.

The evaluation results having been made by the trained evaluation model 210 are transmitted to the central processing unit 121 by the evaluation-result output program 135C, and, if necessary, are displayed in the display 142 and/or are printed through a printer (not illustrated).

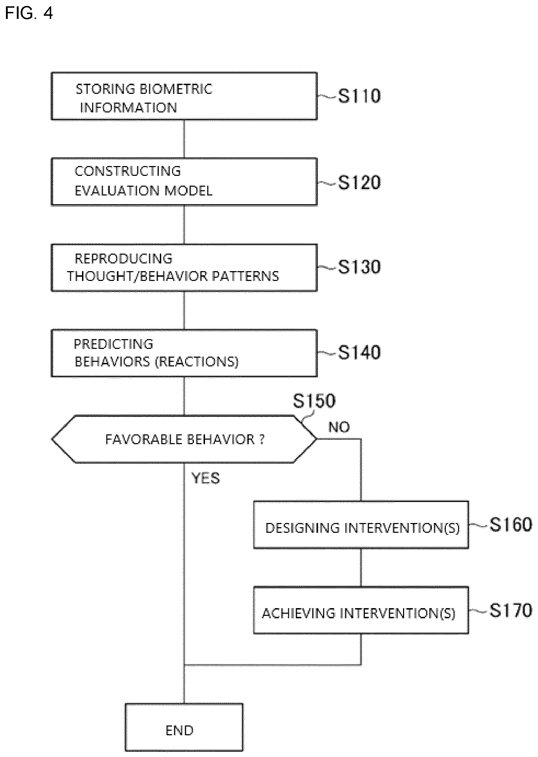

FIG. 4 is a flowchart showing the operation of the portable wireless communication device 100. The operation of the portable wireless communication device 100 is explained hereinbelow with reference to FIG. 4.

First, biometric information of a target person including through patterns and behavior patterns and various data about a target person are collected base on past reactions (words and actions) having been made by a target person in response to external stimulation. The biometric information is stored in the first section 132A, and the various data other than the biometric information is stored in the second section 132B (step S110).

The biometric information includes brain pattern data obtained through a brain imaging device (for instance, fMRI (functional Magnetic Resonance Imaging)), EEG (Electroencephalogram), and so on.

The biometric information and other data are transmitted as teacher data to the evaluation-model constructing program 135B by the teacher-data input program 135A. The evaluation-model constructing program 135B constructs the evaluation model by machine-learning (step S120).

Furthermore, the evaluation-model constructing program 135B uses the thus machine-learned evaluation model to thereby reproduce thought patterns and behavior patterns of a target person (step S130).

The thus reproduced thought patterns and behavior patterns of a target person are transmitted to the second program 131B by the evaluation-result output program 135C.

In the second program 131B, current external stimulation or external stimulation anticipated in the near future to a target person are used as inputs to the evaluation model. The reactions (statements and behaviors) that a target person is expected to exhibit upon receiving those external stimulation are predicted through the evaluation model having been already constructed by the evaluation-model constructing program 135B (step S140).

The predicted reactions of a target person are transmitted to the third program 131C through the evaluation-result output program 135C. The third program 131C determines whether a target person's reactions are favorable to the user of the portable wireless communication device 100 (step S150).

If a target person's reactions are deemed favorable (YES in step S150), no intervention measures (described later) are considered, and the operation of the portable wireless communication device 100 concludes.

If the reactions are not favorable (NO in step S150), the evaluation-model constructing program 135B of the third program 131C designs intervention measures to induce modification in a target person's behavior toward a target behavior (behavior favorable for a user of the portable wireless communication device 100) through the already constructed evaluation model (step S160).

Subsequently, the intervention measures are implemented as appropriate (step S170).

The operation of the portable wireless communication device 100 is explained hereinbelow.

The first program 131A stored in the application-storage section 131 acts as a through reproduction system. Specifically, the first program 131A includes a thought reproduction AI (Sub-Belief System AI = SBS-AI) as learning medium to thereby have a function of reproducing thoughts of a target person. The second program 131B includes a behavior prediction AI as learning medium to thereby have a function as a behavior prediction system. The third program 131C includes a behavior modification AI as learning medium to thereby have a function as a behavior modification system.

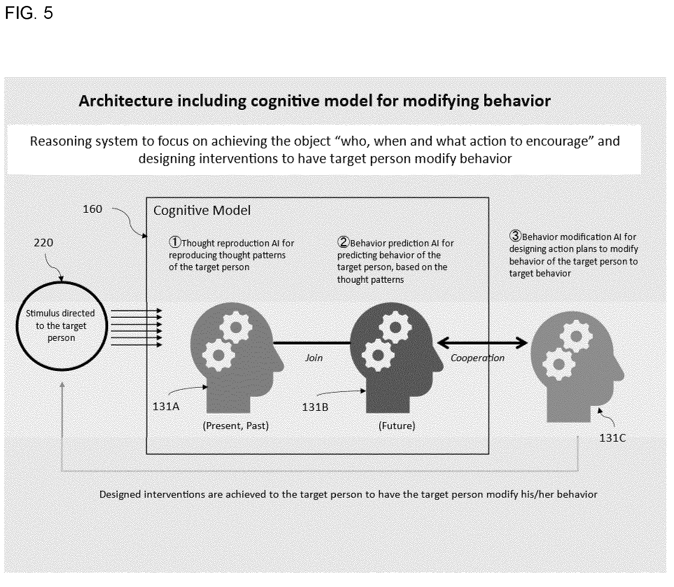

FIG. 5 is a conceptual view showing the collaborative relation among the first to third programs 131A to 131C.

As illustrated in FIG. 5, in brief, the biometric information of a target person (reactions made by a target person in response to external stimulation) and other data about a target person (behavior data of a target person, simulation data to a brain through five senses or words from external environment) are input into the first program 131A (thought reproduction AI) as the input data 220 (see FIG. 3). The first program 131A (thought reproduction AI) constructs a prediction system for each of target persons based on the input data 220. Thought and/or behavior patterns of a target person is reproduced by the prediction system, and the thus reproduced thought and/or behavior patterns are(is) output as the output 230 (see FIG. 3).

The second program 131B (the behavior prediction AI) constitutes a behavior prediction system that receives, as the input 220, the thought/behavior patterns of a target person having been reproduced by the first program 131A, and predicts, based on the thus received thought/behavior patterns, subsequent actions a target person may take, or thought (brain) and behavior patterns a target person may exhibit after a certain period of time passed. Then, the behavior prediction system outputs the prediction as the output 230.

This behavioral prediction forecasts potential behavior patterns of a target person when he/she is in a natural state (a normal situation, namely, not during emergencies such as wartime or disasters).

The behavioral prediction AI is constructed as a pair with each thought-reproduction AI. When a plurality of thought-reproduction Als is parallelized, an equal number of behavioral prediction Als are used to build parallelized structure.

The first program 131A and the second program 131B cooperate with each other to constitute a cognitive model 160 for a target person.

The third program 131C (the behavior modification system) takes, as the input 220, behavioral prediction of a target person having been output by the second program 131B (the behavioral prediction AI). If the predicted future behavior of a target person is deemed undesirable, the third program 131C formulates an intervention plan (an action plan) to modify target person's behavior into desirable behavior (target behavior). This intervention plan is output as the output 230. The thus formulated intervention plan is executed on a target person in a real world to introduce a target person's behavior to change.

Experimental data obtained from a plurality of subjects regarding intervention techniques used in cognitive warfare is used to construct the behavior modification AI.

By allowing the three programs, the first to third programs 131A, 131B, and 131C, to function in coordination, there is formed a cognitive-driven type model which fundamentally differs from a conventional event-driven type model.

Examples of intervention plans formulated by the third program 131C (the behavior modification system) include a variety of methods such as mass media, social media, linguistic guidance, and liminal or subliminal effects through images and videos. In recent years, medical devices directly working to human body have also advanced, and methods of directly intervening an individual's brain have been developed using advanced brain imaging devices such as functional magnetic resonance imaging (fMRI), electroencephalography (EEG), and magnetoencephalography (MEG).

As having been explained so far, in the portable wireless communication device 100, the thought reproduction AI analyzes natural language messages of a target person, and finds hidden intention of a target person. For instance, brain activation state to be measured at real-time by means of functional magnetic resonance imaging (fMRI) is fed back to the thought reproduction AI and vice versa. It is possible to read hidden intention in accordance with a pattern(s) of feedback and/or reaction(s) made by a target person when a signal is interventionally transmitted to a target person.

The behavior prediction AI can provide a hypothesis of a plurality of intentions, as well as a single intention. By recognizing in advance brain activation state in each of intentions by means of fMRI, it would be possible to predict thought and behavior of a target person.

Hereinbelow are explained the first program 131A (thought reproduction AI), the second program 131B (the behavior prediction AI), and the third program 131C (the behavior modification AI).
(A) The first program 131A (thought reproduction system, thought reproduction AI)

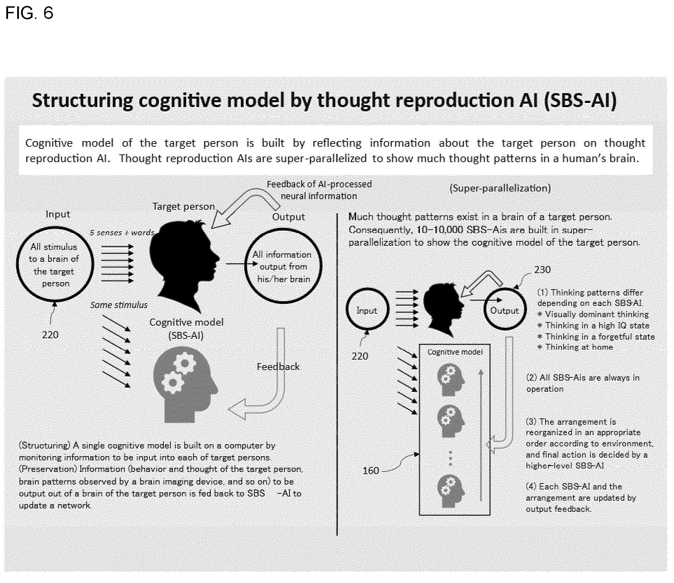

FIG. 6 is a conceptual view showing construction and updating of a cognitive model constructed by the thought reproduction AI (SBS-AI).

The first program 131A constructs "a thought reproduction AI representing brain patterns" as an inference system for each target person.

As shown in FIG. 6, the input 220 to the thought reproduction AI (see FIG. 3) includes the timestamped biometric information of a target person to be transmitted from the first section 132A, and further, behavioral information of a target person to be transmitted from the second section 132B, and stimulus information received by a target person through his/her five senses and language from external environment.

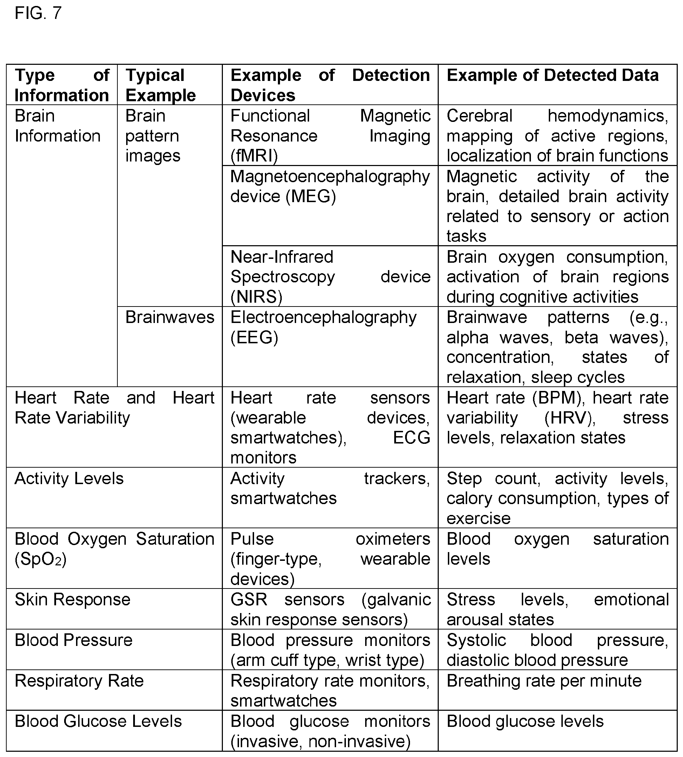

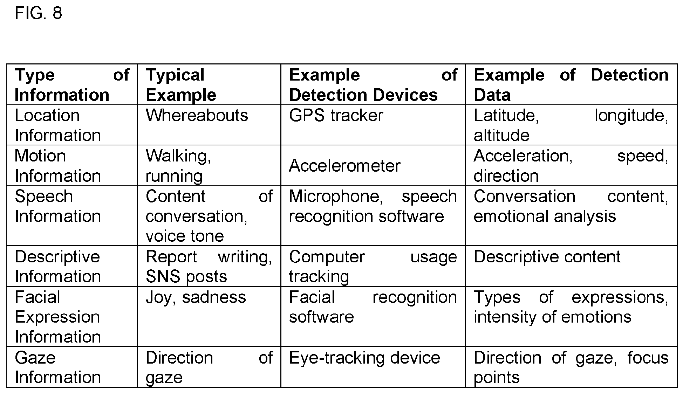

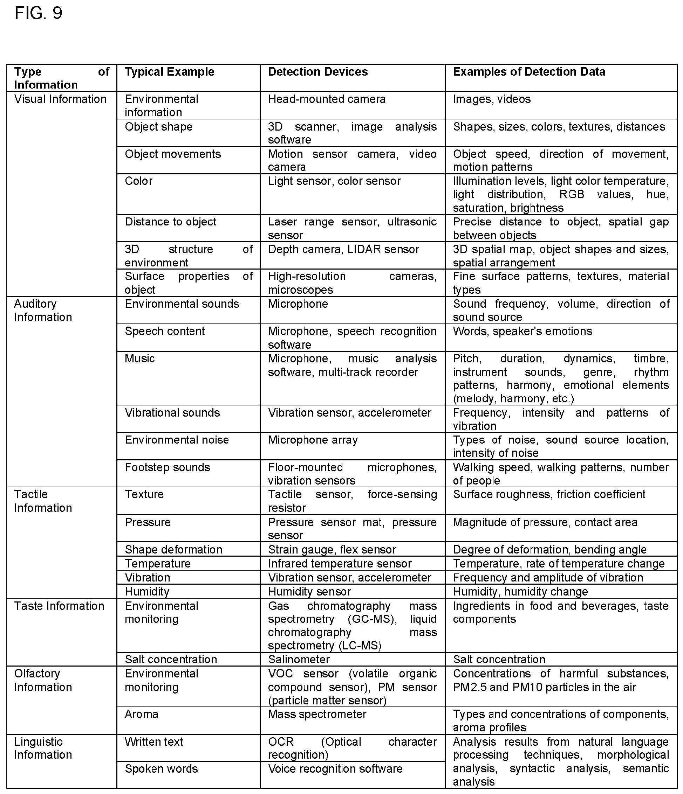

Typical examples of the input 220, examples of a detection device, and examples of detection data are shown in FIGs. 7 to 9. FIG. 7 illustrates an example of biometric information of a target person, FIG. 8 illustrates an example of behavioral information of a target person, and FIG. 9 illustrates an example of stimulus information a target person receives from external environment.

The cognitive model 160 constructed by the first program 131A (thought reproduction AI) has the function of numerically analyzing correlation functions based on the input 220 (input information) to identify thought/behavioral patterns of a target person.

As methods of carrying out numerical analysis, there are principal component analysis used in traditional statistics, support vector machines used in machine learning, and deep learning and large language models (LLM) used for statistical processing with big data. Since analytical methods are rapidly evolving, it is preferable to adopt the most appropriate method of the time.

In the first program 131A (thought reproduction AI), neural networks are used, for example.

By applying a backpropagation method to the thought reproduction AI through the use of the continuously incoming input 220, the thought reproduction AI can be updated. By continuously implementing such feedback, the cognitive model 160 for a target person can be kept up to date.

A plurality of methods may be used to continuously obtain the input 220 (input information).

For example, it is possible to obtain data of a target person through portable wireless communication devices (such as smartphones, wearable devices, and small sensors) used by a target person himself/herself.

As an alternative, it is possible to take data about a target person also through IoT devices and/or smart home devices located close to a target person (home or office).

Alternatively, data about a target person can be obtained by tracking a target person's activities in social media, search behavior through online, and website browsing history.

Additionally, it is possible to directly collect opinions and feedback from a target person through online surveys or in-app popup surveys.

Furthermore, it is possible to directly obtain biometric information of a target person, such as brain waves, muscle activity, and eye movements, by using devices equipped with biosensing technology (for example, Apple Vision Pro^{®}).

In the real world, since all information surrounding a target person, including symbolic information such as language, quasi-symbolic information such as subliminal messages, and non-symbolic information such as biofeedback, generates neural information feedback at various levels of abstraction, cognition of a target person is updated. This phenomenon is known as homeostatic synchronization arising due to interactions between internal representations and external environment, and is inherent to living organisms. Consequently, a method of updating the thought reproduction AI by using backpropagation process with the input 220 (input data) being used as result data can be described as a logic that mimics phenomenon of homeostatic synchronization in computers.

As a result, it is possible to bring the characteristics of a target person's cognition and the characteristics of behavior of the cognitive model 160 closer together. by continuously updating the thought reproduction AI.

In order to build the cognitive model 160 as an ideal one, it is preferable to construct a thought reproduction system at the time of a target person's birth, and input all subsequent biometric, behavioral, and stimulus information.

The portable wireless communication device 100 includes 10 to approximately 10,000 thought reproduction Als embedded therein in parallel within the cognitive model 160 for a single target person. Each of the thought reproduction Als represents a part of diverse brain patterns and personality traits of a target person. For example, thought reproduction Als may represent brain patterns that are visually dominant, are auditorily dominant, are prone to forgetfulness, exhibit high IQ, or reflect strong genetic traits. It is assumed that a target person constantly possesses a plurality of brain patterns with one or more of these brain patterns becoming activated in dependence on environment and situation.

The 10 to approximately 10,000 thought reproduction Als operating in parallel are mutually independent from one another. In each of the thought reproduction Als, weight coefficients of each of neural networks are calculated based on the input 220 (input information), just as in the case of a single thought reproduction AI.

Similarly to a single thought reproduction AI, each thought reproduction AI can be updated through backpropagation based on the continuous input 220.

The activity level of each thought reproduction AI is evaluated based on a distance between information (vector) related to a target person and the output vector output from the thought reproduction AI. The closer the distance between the vectors, the easier the AI is to activate, and the thought reproduction Als with higher activity levels are prioritized. The distance is calculated using Euclidean distance or cosine similarity. The activity level is periodically re-evaluated with new input information, and the order of the thought reproduction Als is updated accordingly.

### (B) The second program 131B (the behavior reproduction system, the behavior reproduction AI)

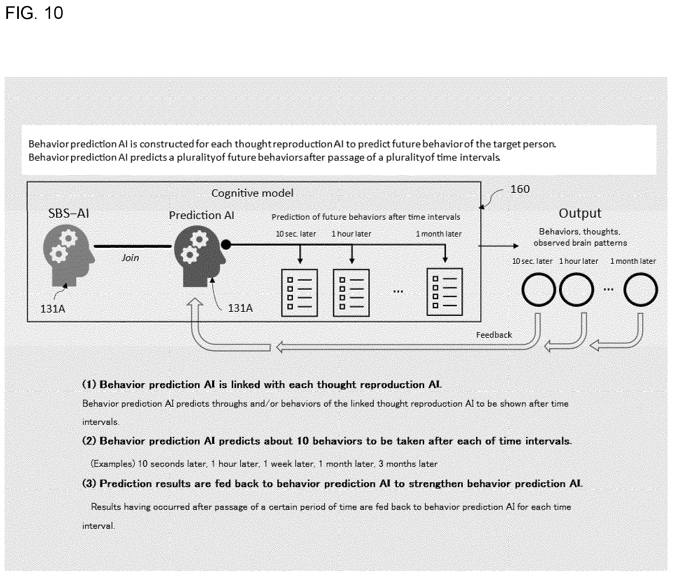

FIG. 10 is a conceptual view of the behavior prediction system constructed by the second program 131B.

The behavior prediction system is equipped with a behavior prediction AI, which is connected with a thought reproduction AI to thereby form a pair with the thought reproduction AI. The behavior prediction AI predicts thoughts and actions of a target person after a time interval T has passed from a given time.

The behavior prediction system takes, as the input 220 (see FIG. 3), thought patterns and/or behavior patterns of a target person to be output from the first program 131A (the thought reproduction AI), and performs numerical analysis of time correlations, based on the input 220, to thereby predict future thoughts and actions of a target person. The numerical analysis may be carried out through statistical and machine learning approaches that handle time correlation functions, or statistical processing using large-scale data (AI), deep learning (DL), or large language models (LLM).

Due to the rapid development of analytical methods, it is desirable to adopt the most appropriate method of the time. For example, neural networks are employed in the behavior prediction system.

The time interval T is not limited to a single, and may be selected in a plurality. When selecting a plurality of time intervals, it is arbitrary to select a length of an interval. For instance, five intervals may be selected including 10 seconds later, 1 hour later, 1 week later, 1 month later, and 3 months later. Generally, a shorter time interval T is suitable for predicting specific actions of a person, while a longer time interval T is suitable for predicting a trend of organization.

By applying backpropagation to the behavior prediction AI based on continuous input information, weight coefficients of a neural network are updated for each future prediction interval T, and thereby, the behavior prediction system is updated.

The behavior prediction system predicts 10 to about 100 actions expected to happen after a time interval T has passed.

If the number of predicted actions is 10, 10 actions are predicted for each of five time intervals (10 seconds later, 1 hour later, 1 week later, 1 month later, and 3 months later), using a pair of the thought reproduction AI and the behavior prediction AI, for a single target person, resulting in that 50 actions are totally predicted (1×5×10 = 50).

The most appropriate predicted action is selected out of these 50 predicted actions.

For instance, two criteria are used to select the most appropriate predicted action, that is, "Most Likely Course of Action (MLCoA)" and "Most Dangerous Course of Action (MDCoA)."

"Most Likely Course of Action (MLCoA)" may be calculated, for instance, by using a softmax function in output layer to calculate a probability of each action.

"Most Dangerous Course of Action (MDCoA)" may be calculated, for instance, by defining a criterion for quantitatively evaluating danger of each action, creating a database of statistical risk assessments based on past data, and referencing the database to calculate risk of predicted actions.

A behavior prediction AI is constructed for each thought reproduction AI, and the two are connected with each other to thereby generate unique prediction patterns for each thought reproduction AI.

Similarly to the single thought prediction system, backpropagation is applied to each thought prediction AI to update, based on continuously supplied input information.

As mentioned above, each thought prediction AI predicts 10 to 100 actions for each time interval T. A total of 500 actions are predicted (10×5×10).

The most appropriate predicted action is selected out of the 500 actions based on the above-mentioned two criteria, that is, "Most Likely Course of Action (MLCoA)" and "Most Dangerous Course of Action (MDCoA)."

In a viewpoint of analytic philosophy, it is said that "a single person can have possible 10 worlds in 10 minutes in the future".

According to a cognitive model composed solely of the thought reproduction AI, inferences can be made only based on past and present information of a target person. In contrast, in a cognitive model composed of a pair of the thought reproduction AI and the behavior prediction AI, an element of time progression is added to inferences, and hence, results of inferences can be enhanced.

If brain patterns are interpreted as activated areas of brain regions, and thought patterns are interpreted as emotions (or affect), there is found a correlation between the two patterns, as described below.

| Brain Patterns (Activated Areas) | Thought Patterns (Emotions) |
|---|---|
| Frontal lobe (especially prefrontal cortex) | Anger |
| Insular cortex, anterior cingulate cortex | Disgust |
| Posterior cingulate cortex | Jealousy |
| Temporal lobe (especially amygdala) | Fear |
| Anterior cingulate cortex, nucleus accumbens | Happiness |
| Ventral striatum, inferior frontal gyrus | Desire |
| Medial prefrontal cortex | Pride |
| Posterior cingulate cortex, temporal lobe | Sadness |
| Dorsomedial prefrontal cortex | Shame |

Furthermore, there are following examples of visuals for evoking the above-mentioned thought patterns (emotions).

| Thought Patterns (Emotions) | Example Visuals |
|---|---|
| Anger | Visuals showing injustice or unfair behavior |
| Disgust | Images of unclean or unpleasant scenes (e.g., images of rotting food) |
| Jealousy | Scenes of a rival succeeding or a romantic partner interacting closely with someone else |
| Fear | Scenes from horror movies or dangerous situations |
| Happiness | Visuals of enjoyable events or cute actions of children and animals |
| Desire | Romantic or sexually appealing visuals |
| Pride | Visuals of personal achievements or being praised |
| Sadness | Scenes of tragic events or heartbreak |
| Shame | Visuals of failures in public or embarrassing situations |

### (C) The third program 131C (the behavior modification system, the behavior modification AI)

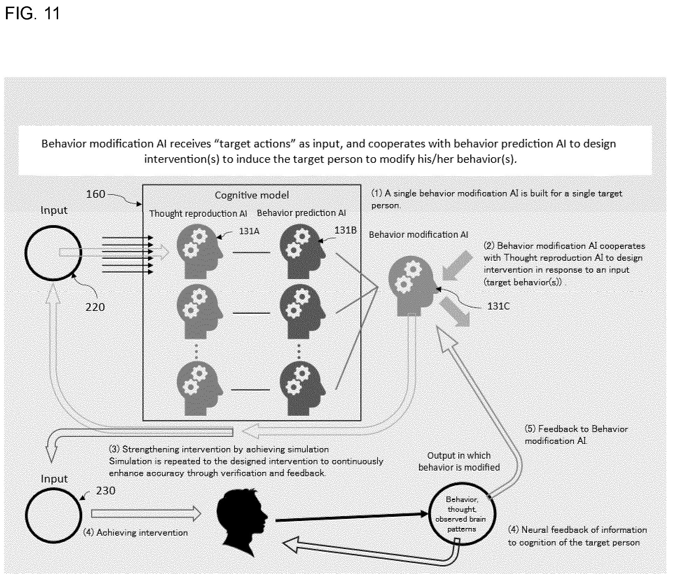

FIG. 11 is a conceptual view of the behavior modification system (the behavior modification AI) constructed by the third program 131C.

The behavior modification system works as an inference system designed to create interventions that lead a specific target person to perform a desired action (target behavior) after the thought reproduction system and the behavior prediction system determined target actions to be performed by the specific target person after a predetermined period of time has passed.

One behavior modification system is built for each target person. In other words, one behavior modification AI is constructed for each cognitive model of a target person, and is not parallelized unlike the thought reproduction AI or the behavior prediction AI.

As input information used for constructing the behavior modification AI, there is used experimental data taken from tests where various intervention actions applied to cognitive warfare were conducted on subjects. This experimental data includes stimulation information that influenced on the subjects, their behavior after receiving the stimulation, and their biometric information.

Intervention actions include mass media, social media (SNS), linguistic guidance, liminal and subliminal effects through images and videos, and the use of medical devices that directly influence a human's body. Brain imaging devices such as functional magnetic resonance imaging (fMRI), electroencephalography (EEG), and magnetoencephalography (MEG) may also be used to directly intervene in a brain of a target person.

During initial construction of the behavior modification AI, limiting a target person to a specific individual reduces an amount of available data. Consequently, the behavior modification AI is constructed based on broad subject data, and the thus generalized behavior modification AI can then be customized for a specific target person.

By copying the generalized behavior modification AI, it is possible to construct behavior modification Als tailored to each target person.

After the behavior modification AI was put into operation, weight coefficients of the behavior modification AI specialized for a target person are continuously updated using new input information obtained when intervention actions started to be conducted on a target person.

As mentioned above, the behavior modification AI collaborates with the thought reproduction AI and the behavior prediction AI to design interventions for guiding a target person to perform a target action(s).

The behavior modification AI receives, as the input 220 (see FIG. 3), "a target person, target action(s) to be performed by the target person, and a deadline for performing the target action(s)" having been output from the second program 131B (the behavior prediction AI), and designs intervention strategies to prompt a target person to perform the target action(s). The behavior modification AI outputs the the thus designed intervention strategies as the output 230.

The intervention strategies designed by the behavior modification AI are not limited to one; but a plurality of strategies may be designed in dependence on circumstances of a target person. Thus, the interventions are designed and the simulation of the interventions are performed concurrently for approximately 10 to 100 plans.

As the output 230 from the behavior modification AI, the intervention strategies include policies, specific actions, and the timing of implementing the interventions.

The intervention strategies designed by the behavior modification AI are placed as input information in a simulation environment constructed by the thought reproduction AI and the behavior prediction AI, where the interventions strategies are tested and validated. By repeating trials and validations, the intervention strategies for a target person are optimized.

Then, the intervention actions are implemented on a target person, encouraging the target person to shift from natural behavior to target behavior. When the intervention actions are conducted, feedback of neural information occurs at all abstraction levels in symbolic, quasi-symbolic, and non-symbolic information in a target person, resulting in that cognition of a target person is modified (homeostasis synchronization).

By continuously implementing the intervention actions, cognitive functions of a target person in real world gradually align with the behavior (target behavior) designed by the cognitive model 160 of the behavior modification introduction system 300.

### (Intervention Delivery System)

However, there are many challenges in implementing intervention strategies. In particular, when interventions are conducted for a target person, existing methods have limitations.

For example, in interventions targeting a wide range of target persons (target person group) through mass media, since each target person has different brain patterns and biological information, it is not possible to expect to yield uniform intervention effects, if the same method is applied to all target persons.

In addition, while a brain imaging device is effective in interventions to be applied for a single target person, it requires consent of the target person for the use of such devices. Furthermore, analyzing large amounts of data demands enormous computational resources, making it impractical.

To address these issues, the behavior modification introduction system 300 in accordance with the present embodiment adopts an intervention delivery system that enables scalability to accommodate large numbers of target persons.

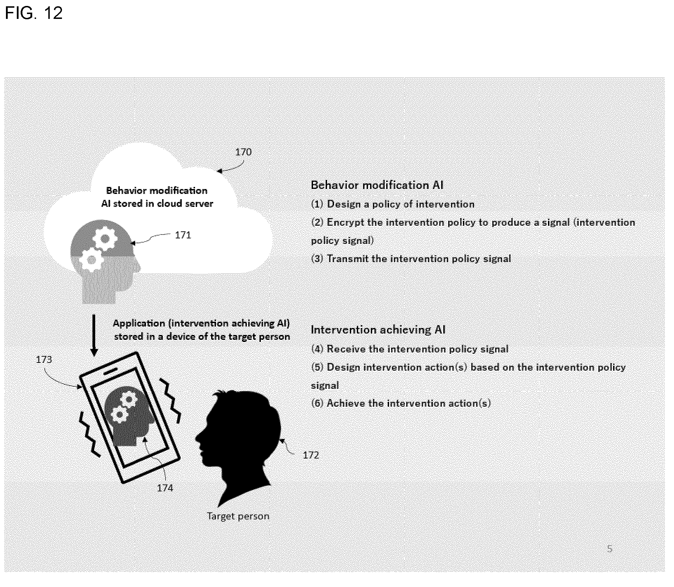

FIG. 12 is a conceptual view of the intervention delivery system.

In the intervention delivery system, intervention strategies are grouped into intervention policy signals having high-level abstraction, and specific intervention actions.

By separating intervention strategies into intervention policy signals and intervention actions, it is accordingly possible to separate Als used for behavior modification of target persons, as well.

For example, a behavior modification AI stored in a cloud server may be responsible for designing intervention policy signals, while an intervention execution AI presented in an application on a device (such as a mobile phone) owned by a target person may be responsible for generating and implementing intervention actions.

Specifically, as illustrated in FIG. 12, the intervention delivery system comprises a behavior modification AI 171 on a cloud server 170 for generating intervention policy signals, and an intervention execution AI 174 on an application stored in a portable wireless receiving device (e.g., smartphone) 173 possessed by a target person 172.

The behavior modification AI 171 (third program 131C) on the cloud server 170 collaborates with both the first program 131A (thought reproduction AI) and the second program 131B (the behavior prediction AI) to design intervention policies for modifying behavior of the target person 172. The intervention policy is encrypted and compressed, resulting in a lightweight intervention policy signal of approximately 8 bytes in size, similar to MIDI data. This intervention policy signal contains information having relatively high-level abstraction, ensuring that a data size remains minimal.

The intervention policy signal is transmitted to the portable wireless communication device 173 possessed by the target person 172 via existing information communication infrastructure (Internet).

Upon receipt of the intervention policy signal from the behavior modification AI 171, the intervention execution AI 174 stored in the portable wireless communication device 173 formulates and executes intervention actions customized for each target person, based on the received signal.

Examples of such intervention actions include rewriting text in chat messages, issuing fake earthquake alerts or other disaster warnings, and displaying subliminal images on a display of the portable wireless communication device 173.

Additionally, it is also possible to obtain information about the target person 172 (such as operation history, browsing history, center information, and so on) through the application (intervention execution AI 174) stored in the portable wireless communication device 173.

For instance, in the case that the above-mentioned intervention delivery system is applied to a marketing domain, the intervention policy may consist of an intervention policy signal such as "campaign page of our product is exposed to potential customers within one hour," and an intervention action necessary to achieve this goal, such as " a push notification is displayed on a display of the potential customer's portable wireless communication device (e.g., smartphone)."

In the case that the intervention delivery system is applied to a military domain, the intervention policy consists of an intervention policy signal such as " the enemy nation's leader is dissuaded within six months from launching military invasion" and an intervention action necessary to achieve this goal, such as "an official announcement is made at an international organization to shape global public opinion that military invasion cannot be justified."

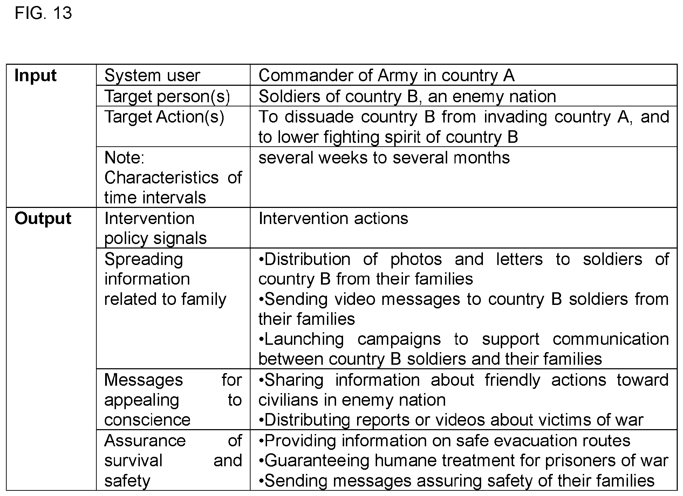

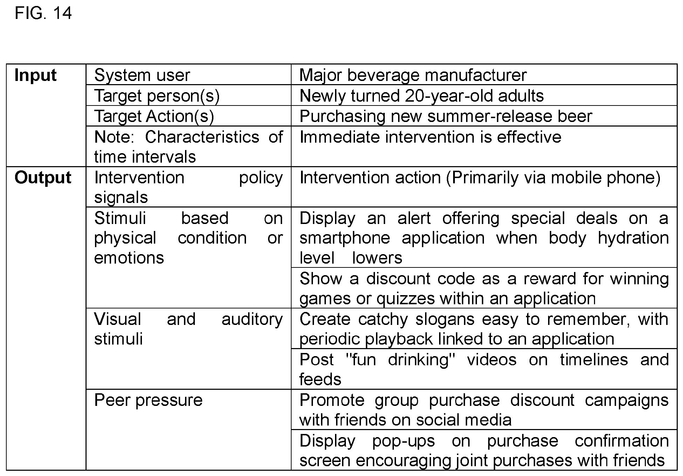

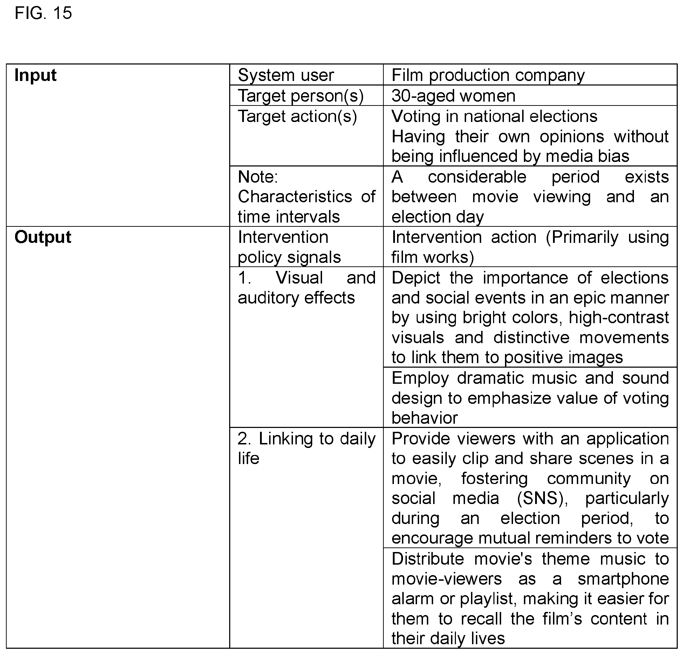

FIGs. 13, 14, and 15 illustrate examples of input and output (intervention policy signals and intervention actions) in a security domain, a marketing domain, and an entertainment domain, respectively.

### (Examples)

Hereinbelow are explained examples to which the behavior modification introduction system 300 is specifically applied.

### (First Example)

In the first example, the behavior modification introduction system 300 acts as a cognitive warfare defense system.

A target person(s) in the cognitive warfare defense system may include, for example, a leader(s) of an enemy nation(s), enemy military personnel, enemy civilians, and citizens of other countries (singular or plural) who form a core of international public opinion.

When sufficient computational resources are available, it is preferable to build cognitive models for entire population. However, if computational resources are limited, the target person(s) is(are) restricted to one or a few persons.

For example, as an enemy leader(s), a target person(s) may be limited to the prime minister of an enemy country and several close aides who influence the prime minister's decisions. For enemy military personnel, a target person(s) may be limited to a commander of enemy forces and his aides influencing the commander's decisions. In the case of enemy civilians or citizens of other countries who shape international public opinion, cognitive models can be constructed collectively for people belonging to a specific age group, residents of a particular region, or citizens of a specific country.

For instance, in the case that a target action against an enemy country is to prevent outbreak of war, an intervention policy may be designed to make an enemy leader(s) feel that initiating a war will jeopardize his/their position(s). For enemy military personnel, an intervention policy may be designed to make them feel that victory is unattainable from the perspective of force and logistics. For enemy civilians, an intervention policy may be designed to make them feel that their lives will become more difficult if war breaks out.

Additionally, for citizens of other countries who shape international public opinion, an intervention policy may be designed to encourage them to raise their voices in condemning enemy nation's declaration of war.

### (Second Example)

In the second example, the behavior modification introduction system 300 acts as a marketing system.

In the marketing system, it is the goal to understand consumer behavior, and expand influence based on the understanding. The behavior modification induction system 300 makes it possible to replicate consumers' decision-making processes, preferences, needs, and behavioral patterns, and design and implement a marketing strategy personalized for target persons (potential customers).

In the marketing strategy, the following steps are implemented, for instance.

First, the cognitive model 160 (see FIG. 5) is constructed for each consumer (a target person).

Specifically, the individual cognitive model for each consumer is constructed, based on biological information such as brain patterns, as well as data related to purchasing behavior, including a consumer's past purchase history, online activity trails, and reactions or expressed opinions on social media (SNS).

The cognitive model 160 makes it possible to infer what kind of cognitive biases consumers have toward specific products or services, and which factors promote or inhibit purchasing motivation.

Second, a personalized marketing strategy is designed.

Specifically, by utilizing the constructed cognitive model 160, marketing messages and/or promotional activities optimized for consumer's current needs and future desires, as indicated by the behavior prediction AI (second program 131B), are planned. Thus, deep psychology and latent needs of consumers are reproduced in a marketing domain, enabling to create effective approaches tailored to them, and further, enabling direct contribution to enhancing brand value of a company and increasing sales.

### (Third Example)

In the third example, the behavior modification introduction system 300 acts as an entertainment system.

By applying the behavior modification inducement system 300 to an entertainment field, it is possible to provide personalized experiences tailored to preferences and behavior patterns of each of target persons in various ranges including movie viewers, game and music enthusiasts, and more.

In the entertainment system, the following steps are implemented, for instance.

Firstly, the cognitive model 160 is constructed for each target person such as a viewer and so on.

Specifically, an individual cognitive model 160 for each target person is constructed based on biological information such as brain patterns, heart rate, skin conductivity, brain waves, and pupil responses observed when he/she watches a work (such as a movie and a stage), as well as his/her past movie viewing history, game play records, and music streaming information. The cognitive model 160 makes it possible to infer which genres and/or themes each target person is particularly interested in, what types of stories or gameplays they prefer, and what moves him/her emotionally.

Secondly, personalized content is recommended.

Using the constructed cognitive model 160, the most suitable movies, games, music, and other contents are recommended to each target person. Furthermore, recommendations can be made at a detailed level, such as a method of visual representation, progression of a game's story, or a tone of music.

Thirdly, experiences of interactive contents are customized based on the viewer's cognitive model.

For example, scenes on movies that previously elicited strong emotional reactions from a viewer are analyzed to thereby recommend contents which evoke similar emotions, or events in-a game are adjusted according to a player's preferences.

Fourthly, user's experiences are continuously optimized.

By incorporating the feedback from a target person and new behavioral data about a target person into the cognitive model 160, experiences of a target person is continuously optimized. This allows for quick adaptation to changing preferences and new trends of a target person, consistently providing the best entertainment experiences.

Thus, by applying the behavior modification inducement system 300 to an entertainment field, an entertainment industry can offer more personalized and immersive experiences, significantly enhancing satisfaction and loyalty of viewers and players. Additionally, this approach is expected to promote discovery of new contents and expand entertainment consumption.

In order to make it possible to readily understand the behavior modification induction system 300 in accordance with the present embodiment, specific examples are detailed hereinbelow.

### (Specific Example 1)

### 1. Target person

It is supposed that an individual X is a target person.

### 2. User of the behavior modification induction system 300

It is supposed that a friend or a family of the individual X uses the system 300 to attempt to modify behavior of the individual X.

### 3. Teacher data

The following input data (stimulation) and output data (reaction) are used as the teacher data 200 (see FIG. 3).

The input data (stimulus) to be directed to the individual X is as follows.
(A) Showing frightening images (such as in movies or television)
(B) Telling scary stories (such as reading thriller novels to the individual X)

The reactions (output data) shown by the individual X in response to these input stimuli were as follows:
(1) Heart rate increases
(2) Cold sweat
(3) Attempt to flee

These teacher data 200 are transmitted to the evaluation model generation program 135B through the teacher data input program 135A of the first program 131A along with other biometric information about the individual X. The evaluation model generation program 135B generates an evaluation model 210 through machine learning (see step S120 in FIG. 4).

### 4. Behavior Prediction by the behavior prediction AI

After the evaluation model 210 undergoes training, the second program 131B (the behavior prediction AI) predicts behavior of the individual X to be made in response to real-world stimuli.

When the individual X encounters the following stimuli (a) to (c), the second program 131B (the behavior prediction AI) predicts the following behaviors.

### (a) The individual X listens to a ghost story

The behavior of the individual X predicted by the behavior prediction AI in response to this stimulus is "no reaction" or "slight increase in heart rate."

### (b) The individual X mistakes something for a ghost while walking at night

The behavior of the individual X predicted by the behavior prediction AI in response to this stimulus is "falling into a state of mental shutdown (mind goes blank)."

### (c) A snake suddenly appears in front of the individual X while walking in mountains

The behavior of the individual X predicted by the behavior prediction AI in response to this stimulus is "attempt to flee, but unable to move due to fear" or "collapse from shock."

### 5. Behavior modification actions by the behavior modification AI

In response to the behavior predictions having been output by the behavior prediction AI, the third program 131C (the behavior modification system) formulates behavior modification actions to alter the individual X's behavior.

There are two directions (A) and (B) for behavior modification.
(A) Behavior modification to weaken emotions (affective response) caused by stimulus (weakening behavior modification)
(B) Behavior modification to replace emotions (affective response) caused by stimulus with other emotions (replacing behavior modification)

The third program 131C (the behavior modification system) designs one of (A) and (B), or both of (A) and (B).

### (5.1) For instance, it is supposed that the individual X has to go out at night.

In this case, a weakening behavior modification action formulated by the behavior modification AI (behavior modification action in response to "the individual X mistakes something for a ghost") might be as follows:
(A) Provide the individual X with a helmet equipped with a powerful light and have him/her wear the helmet while walking, illuminating a path ahead.

If a path ahead is brightly lit, the likelihood of "the individual X mistakes something for a ghost" is significantly reduced.

The third program 131C (the behavior modification system) notifies the behavior modification action to the user of the behavior modification guidance system 300 (a friend or family member of the individual X) through an email, or displays the behavior modification action on a screen. This allows the user of the behavior modification guidance system 300 to purchase a helmet with a powerful light, and make the individual X have the helmet.

Alternatively, if time allows, the third program 131C (the behavior modification system) may automatically order a light-equipped helmet on an online store (such as Amazon), and have the ordered helmet delivered directly to the user of the behavior modification guidance system 300 or directly to the individual X via express delivery.

(B) Continue a conversation between the individual X and an automated voice through the individual X's portable wireless communication device. Specifically, the third program 131C (the behavior modification system) analyzes words spoken by the individual X, generates appropriate responsive words through an automated voice system, and then, makes the responsive words speak through the individual X's portable wireless communication device.

Thus, the individual X is kept focused on the conversation, leaving no room to mistake something for a ghost.

Alternatively, an icon may appear on a display of the individual X's mobile phone, allowing the individual X to engage in a conversation with the icon.

(C) The third program 131C (the behavior modification system) utilizes a map function on the individual X's portable wireless communication device to find a well-lit route to a destination, and guides the individual X to the destination through the portable wireless communication device.

Avoiding dark paths reduces a likelihood of misperceptions.

Alternatively, instead of selecting a well-lit route, a route with pedestrian traffic may be selected.

The individual X can have a sense of security by walking a route with pedestrian traffic, resulting in reduction of a likelihood of misperceptions caused by anxiety.

The replacing (substitutive) behavior modification actions formulated by the behavior modification AI may include the following examples.

(D) Streaming beautiful nighttime cityscapes or soothing music to the individual X's portable wireless communication device to elicit positive emotions.

This not only alleviates the individual X's sense of fear, but also draws out positive emotions by allowing the individual X to enjoy beautiful scenery and pleasant music.

(E) Encouraging video calls with friends or family through the individual X's portable wireless communication device to provide a sense of security and foster a sense of human connection and solidarity.

In situations where the individual X would typically feel fear, this action promotes awareness of connections with friends and family, providing the individual X with reassurance.

(F) Displaying photos or videos of enjoyable past memories on a display of the individual X's portable wireless communication device to elicit positive emotions.

By replaying pleasant past memories in situations where the individual X would typically feel fear, the sense of fear is overwritten with positive emotions.

### (5.2) For instance, it is supposed that the individual X goes hiking.

In this case, the weakening behavior modification actions formulated by the behavior modification AI (behavior modification actions for a scenario of "suddenly encountering a snake") may include the followings.
(A) Recommending the individual X to wear sturdy (highly resistant to impact) boots.

Since a snake tends to bite on feet, it is possible to provide the individual X with a sense of security by protecting his/her feet.

The third program 131C (the behavior modification system) notifies the behavior modification action to the user of the behavior modification induction system 300 (friends or family of the individual X) through an e-mail, for instance, and/or displays the behavior modification action on a screen. This allows the user of the behavior modification induction system 300 to purchase boots and have the individual X wear them.

Alternatively, if time permits, the third program 131C (the behavior modification system) may automatically order boots on an online retail website (such as Amazon) and have them delivered by express shipping to either the user of the behavior modification guidance system 300 or directly to the individual X.

(B) Having the individual X wear clothing which can prevent heat diffusion.

Since snakes detect prey by sensing heat with their tongues, preventing heat emission reduces a likelihood of detection.

The third program 131C (the behavior modification system) notifies the behavior modification action to the user of the behavior modification induction system 300 (friends or family of the individual X) through an e-mail, for instance, and/or displays the behavior modification action on a screen. This allows the user of the behavior modification induction system 300 to purchase heat-diffusion prevention clothing and have the individual X wear it.

Alternatively, similarly to the case (A), the third program 131C (the behavior modification system) may automatically order the clothing on an online retail website.

(C) The third program 131C (the behavior modification system) provides a hint such as "snakes are not scary" visually or audibly to the individual X through the display 142 or the speaker 145 of the individual X's portable wireless communication device.

Since snakes will flee unless attacked in most cases even if snakes and a human encounter each other, the action of giving a hint reduces unnecessary fear to be felt by the individual X.

The substitutive behavior modification actions formulated by the behavior modification AI may include the followings.

(D) Positive information about beautiful mountain scenery or local flora and fauna is(are) displayed on a screen of the individual X's portable wireless communication device to enable the individual X to walk enjoying them.

By overwriting a fear against snakes with beautiful scenery and interesting information about flora and fauna, a fear against snakes can be mitigated, allowing the individual X to enjoy hiking.

For example, the user of the behavior modification induction system 300 can send instructions to the third program 131C (the behavior modification system) through the individual X's portable wireless communication device to display the above-mentioned positive information.

(E) Playing audio guides emphasizing health benefits and relaxation effects of hiking through the individual X's portable wireless communication device.

Fear against snakes is overwritten with positive effects of health and relaxation.

For instance, the user of the behavior modification induction system 300 can transmit instructions to the third program 131C (the behavior modification system) through the individual X's portable wireless communication device to thereby play the above-mentioned audio.

(F) Displaying photos or videos of enjoyable past hiking or nature exploration experiences on a display of the individual X's portable wireless communication device.

By replaying pleasant hiking or nature exploration memories in situations where the individual X would typically feel fear, fear against snakes is overwritten with positive emotions.

For instance, the user of the behavior modification induction system 300 can transmit instructions to the third program 131C (the behavior modification system) through the individual X's portable wireless communication device to display those images or videos.

### (Specific Example 2)

### 1. Target person

It is supposed that the president P of a country R is a target person.

### 2. User of the behavior modification induction system 300

It is supposed that a government of a country F which is in conflict with the country R uses the system 300 to attempt to modify behavior of the president P.

### 3. Teacher data

The following input data (stimulus) and output data (reaction) are used as the teacher data 200 (see FIG. 3).

The input data (stimulus) to be directed to the president P is as follows.
(A) The domestic economy is sluggish (a price of crude oil, which is a source for acquiring foreign currency for the country R, has fallen, resulting in a lack of foreign currency inflow into the country R).
(B) A large oil field has been newly discovered in another country, or a shale revolution has occurred in another country (this further lowers crude oil price, making the economy of the country R, which relies on crude oil, even more difficult).

Reactions (output Data) demonstrated by the president P in response to the above-mentioned input data (stimuli) are as follows.
(1) To divert public dissatisfaction due to sluggish domestic economy, military activities are conducted abroad (such as annexing a territory of another country or intervening militarily in conflicts of other countries)
(2) Strengthening domestic intelligence networks (cracking down on dissidents in the country R)
(3) Strengthening military power

### 4. Behavioral prediction made by the behavior prediction AI

After the evaluation model 210 undergoes training, the second program 131B (the behavior prediction AI) predicts how the target person will act in response to real-world stimuli.

When the target person encounters the following stimuli, the second program 131B (the behavior prediction AI) makes the following behavioral predictions.
(a) A country S, which has maintained neutrality toward both the country R and its adversary country F, abandons its neutral policy and joins military alliance N (for example, NATO), to which the country F belongs.

The behavior of the target person predicted by the behavior prediction AI in response to this stimulus is "to prevent further expansion of the military alliance **N,** the target person will invade a neighboring country U, which has not yet joined the military alliance N, and annex the country U into the country R."

### 5. Behavior modification action(s) made by the behavior modification AI

In response to the predicted behavior of the target person (invasion to the neighboring country U) having been output by the behavior prediction AI, the third program 131C (the behavior modification system) formulates behavior modification actions to alter the target person's behavior.

As described earlier, the third program 131C (the behavior modification system) formulates both weakening behavior modification actions and substitutional behavior modification actions.

The weakening behavior modification actions formulated by the behavior modification AI may include the followings, for instance.
(A) Requesting leaders of various countries to strengthen military support for the country U.
   Specifically, the third program 131C (the behavior modification system) encourages the country F, that is, the user of the behavior modification induction system 300, to issue statements or send emails to leaders of other countries.
(B) Promoting international public opinion through the media.
   For example, the third program 131C (the behavior modification system) encourages the country F, that is, the user of the behavior modification induction system 300, to provide information about the country R's movements and intentions to media around the world, and to prompt the media to publish articles protesting and criticizing the country R's plans to invade the country U.
(C) Announcing economic sanctions (such as boycott of oil from the country R). For example, the third program 131C (the behavior modification system) encourages the country F, that is, the user of the behavior modification induction system 300, to announce economic sanctions or notify world leaders to participate in the economic sanctions.
(D) Emphasizing support for the country U by nations hostile to the country R, and issuing statements fostering international solidarity.

This will make the target person feel internationally isolated and fear diplomatic failure, leading to abandonment of invasion to the country U.

For example, the third program 131C (the behavior modification system) encourages the country F, that is, the user of this behavior modification induction system 300, to issue statements to world leaders for showing the above-mentioned policy and requesting cooperation to the policy. Furthermore, the third program 131C encourages the country F to request the media to issue an article for appealing the necessity of such programs.

(E) Proposing international economic cooperation programs to support economic recovery of the country R.

If the country R's economy recovers, the need to invade the country U to divert public attention abroad will gradually diminish.

For example, the third program 131C (the behavior modification system) encourages the country F, that is, the user of the behavior modification induction system 300, to present such policies, and call on other countries for cooperation. Additionally, the country F is encouraged to request the media to publish articles appealing for the necessity of such programs.

(F) Conducting information warfare to suppress anti-government activities in the country R, and emphasize domestic political stability.

If domestic political stability improves, the necessity to invade the country U to divert public attention abroad will diminish.

For example, the third program 131C (the behavior modification system) encourages the country F, that is, the user of the behavior modification induction system 300, to propose such policies and call on other countries (allied nations) for cooperation.

The substitutional behavioral modification actions formulated by the behavioral modification AI may include the followings, for examples.

(G) Providing international conferences or negotiation platforms that can highlight diplomatic success for the target person, evoking a sense of happiness derived from the diplomatic success.

By emphasizing international cooperation and support for the country R, the behavioral modification AI has the target person feel a sense of diplomatic success, thereby reducing the target person's motivation for invasion.

For instance, the third program, 131C (the behavior modification system) prompts the country F, that is, the user of the behavioral modification induction system 300, to notify world leaders about hosting an international conference or to encourage the media to publish articles advocating for the necessity of such international conferences.

(H) Proposing economic policies or infrastructure projects that could improve an approval rating of the target person (the president P) in the country R, drawing out of the target person a sense of happiness derived from increased support.

By emphasizing domestic economic stability or infrastructure development and garnering public support, the behavior modification induction system aims to eliminate the necessity of invasion.

For example, the third program, 131C (the behavior modification system) could present such policies or projects to the country F, that is, the user of the behavior modification induction system 300, encouraging the country F to communicate these proposals to the country R.

(I) Promoting cultural or sports events that would bring acclaim to the target person both domestically and internationally, fostering a positive international image of the target person.

Through international sports events or cultural exchanges, the behavior modification system aims to evoke positive international images and admiration for the target person, reducing the necessity for invasion.

For instance, the third program, 131C (the behavior modification system) may suggest plans for such sport events to the country F, that is, the user of the behavior modification induction system 300, and prompt the country F to notify world leaders about participation to the events or request the media to publish articles advocating for the necessity of such sport events.

(J) Providing new diplomatic initiatives to make the target person feel a sense of international success.

By having the target person feel international support and emphasizing diplomatic achievements, the behavior modification system seeks to alleviate the target person's fear of losing power, and dissuade the target person from invasion to the country U.

For instance, the third program, 131C (the behavior modification system) could prompt the country F, that is, the user of the behavior modification induction system 300, to notify global leaders about hosting international conferences to present the diplomatic initiatives to the country R or encourage the media to publish articles advocating the necessity of such international conferences.

(K) Providing success stories and/or positive data highlighting economic growth and domestic stability of the country R, creating a sense of economic security.

By alleviating economic fears and further by overwriting such economic fears with data about domestic stability and growth, the behavior modification system aims to deter aggression to the country U.

For example, the third program, 131C (the behavior modification system) might present such examples and/or data to the country F, that is, the user of the behavior modification induction system 300, encouraging the country F to relay them to the target person.

(L) Offering success stories of cultural exchanges and/or sport events that would bring international acclaim to the target person to thereby emphasize a positive international image of the target person.

Through international success stories, the behavior modification system aims to evoke positive emotions from the target person and reduce fears of the target person, thus discouraging invasion to the country U.

For example, the third program, 131C (the behavior modification system) could provide emails and/or videos showcasing these examples to the country F, that is, the user of the behavior modification induction system 300, prompting the country F to distribute them to the media in other countries.

### (Specific Example 3)

### 1. Target person

It is supposed that a heavy viewer of videos (moving pictures) on portable wireless communication devices (smartphones) who rarely visits movie theaters is a target person.

### 2. User of the behavior modification induction system 300

It is supposed that a friend or a family member of the target person uses the system 300 to attempt to modify behavior of the target person.

### 3. Teacher data

The following input data (stimulus) and output data (reaction) are used as the teacher data 200 (see FIG. 3).

The input data (stimulus) provided to the target person are as follows:
(A) Showing the target person his/her favorite short video clips.
(B) Playing relaxing music while the target person is watching the videos.
(C) Providing easily enjoyable video contents.

The responses (output data) demonstrated by the target person to the above-identified stimulus were as follows.
(1) Smiling
(2) Taking a relaxed posture
(3) Feeling a sense of comfort

### 4. Behavior prediction made by the behavior prediction AI

After the evaluation model 210 has been trained, the second program 131B (the behavior prediction AI) predicts how the target person will behave in response to real-world stimuli.

The second program 131B (the behavior prediction AI) predicts the behavior to be demonstrated by the target person when he/she encounters the following stimuli, as follows.
(a) Showing the target person an action movie trailer which can excites the target person
   The behavior of the target person predicted by the behavior prediction AI in response to the stimulation (a) is "show a thrilled expression" or "lean forward in his/her posture."
(b) A friend of the target person makes a plan to go to a movie theater with the target person.
   The behavior of the target person predicted by the behavior prediction AI in response to the stimulation (b) is "He/she actively expresses an interest in going to a movie theater."
(c) Showing the target person information about special events to be held at a movie theater.

The behavior of the target person predicted by the behavior prediction AI in response to the stimulation (c) is "He/she decides to go to a movie theater" or "He/she starts preparing to go to a movie theater."

### 5. Behavior modification actions made by the behavior modification AI

In response to the predicted behavior having been output by the behavior prediction AI, the third program 131C (the behavior modification system) formulates behavior modification actions to modify the target person's behavior.

As mentioned earlier, the third program 131C (the behavior modification system) designs both weakening behavior modification actions and substitution behavior modification actions.

(5.1) For instance, it is supposed that the target person lazily prefers watching videos (moving pictures) on his/her portable wireless communication device (smartphone) at home.

In such a case, the behavior modification AI formulates weakening behavior modification actions as follows.
(A) Limiting video viewing time on his/her portable wireless communication device.

For instance, the third program 131C (the behavior modification system) transmits a reminder to the user of the behavior modification induction system 300 about limiting the target person's video viewing time, and further, displays a content of the reminder on a screen of the target person's portable wireless communication device, and/or play automated messages periodically through the portable wireless communication device.

Alternatively, the user of the behavior modification induction system 300 can remotely control the target person's portable wireless communication devices to mandatorily limit a time in which the target person is allowed to watch videos.

(B) Informing the target person of health impacts caused by prolonged viewing. For instance, the third program 131C (the behavior modification system) notifies the user of the behavior modification induction system 300 of health impacts, and the user directly informs the target person of the health impacts, or display messages on the target person's smartphone screen, or play automated messages periodically.

(C) Sending a message to encourage breaks after a set period of video viewing.

For instance, the third program 131C (the behavior modification system) notifies the user of the behavior modification induction system 300 of necessity of breaks, and the user encourages the target person to have breaks. Alternatively, the user may display such messages on a screen of the target person's portable wireless communication device, or play automated reminders periodically.

The behavior modification AI formulates substitution behavior modification actions as follows, for instance.

(D) Showing trailers or behind-the-scenes of exciting action movies to thereby have the target person expect to have more impressive experience, if he/she watches the movies at a movie theater.

For instance, the third program 131C (the behavior modification system) display such videos on a display of the target person's portable wireless communication device, replacing video viewing at home with exciting experience of watching at a movie theater.

(E) Notifying the target person of information about special events or limited-time screenings at a movie theater, prompting him/her to plan to go to a movie theater with his/her friend(s).

For instance, the third program 131C (the behavior modification system) notifies the user of the behavior modification induction system 300 of the information, and then, the user provides the information to the target person.

Alternatively, the third program 131C (the behavior modification system) notifies the target person of the information by transmitting e-mail or through a display or a speaker, emphasizing unique movie theater experience to thereby prompt the target person to plan active action, that is, plan going to a movie theater with his/her friend(s).

(F) Displaying photos or videos of enjoyable past movie theater experiences to the target person to rekindle his/her desire to revisit a movie theater.

For instance, the third program 131C (the behavior modification system) shows such visuals on a display of the target person's portable wireless communication device, evoking past enjoyable memories at a movie theater to thereby activate him/her in actions.

(5.2) For instance, it is supposed that the target person decides to visit a movie theater.

In this case, the behavior modification AI formulates weakening behavior modification actions as follows.
(A) Providing an application to easily search and book transportation to a movie theater.
   For instance, the third program 131C (the behavior modification system) could automatically download the application on the portable wireless communication device of the target person even without instructions from the target person and the user to thereby minimize hassle of searching transportation to a movie theater, thereby lowering barriers to going to a movie theater.
(B) Offering discount coupons or special benefits for a movie theater to highlight advantages of visiting a movie theater.
   For instance, the third program 131C (the behavior modification system) could display such discount coupons or special benefits on a display of the portable wireless communication device of the target person. This encourages the target person to make action by having the target person feel profitable if he/she would go to a movie theater.
(C) Providing real-time information about crowd status and screening schedule of movie theaters, enabling the target person to visit at the optimal time.

For example, the third program 131C (the behavior modification system) displays such information on a display of the target person's portable wireless communication device, aiming to ensure a smooth experience at a movie theater and encouraging the target person to make action.

The substitutive behavior modification actions formulated by the behavior modification AI may include the followings.

(D) Highlighting special events and/or interactive experiences available at a movie theater, suggesting unique enjoyment that can be experienced only there.

For instance, the third program 131C (the behavior modification system) displays such information on a display of the target person's portable wireless communication device, aiming to replace home video viewing with a special experience to be obtained in a movie theater.

(E) Proposing making a plan to enjoy movies at a movie theater with a friend(s) or family, emphasizing social connections.

For example, the third program 131C (the behavior modification system) displays such suggestions on a display of the target person's portable wireless communication device. This frames a theater experience as a social event and encourages the target person to be active in actions.

(F) Providing video messages that recall past enjoyable experiences at a movie theater or highlight anticipated new experiences, eliciting a desire to visit a movie theater.

For instance, the third program 131C (the behavior modification system) displays such video messages on a display of the target person's portable wireless communication device to thereby emphasize positive experiences associated with a movie theater and motivate active behavior of the target person.

### (Specific Example 4)

### 1. Target person

It is supposed that the president P of a country R is a target person.

### 2. User of the behavior modification induction system 300

It is supposed that the Japanese government uses the behavior modification induction system 300 to attempt to modify behavior of the president P.

### 3. Teacher data

The following input data (stimulus) and output data (reaction) are used as the teacher data 200 (see FIG. 3).
(A) Presenting materials and records showing that a country U neighboring to the country R has traditionally been a part of the country R's territory.
(B) The president P receives information suggesting that residents of a specific region in neighboring country U were originally citizens of the country R, and are being mistreated by the government of the country U.

The reactions (output data) shown by the target person in response to these input data (stimuli) (A) and (B) were as follows.
(1) The target person (the president P) made up his/her mind to help the residents of a specific region in neighboring country U, and decided to invade the country U.

### 4. Behavioral prediction made by the behavior prediction AI

After the evaluation model 210 has been trained, the second program 131B (the behavior prediction AI) predicts actions the target person will take in response to real-world stimuli.

When the target person encounters the following stimuli, the second program 131B (the behavior prediction AI) predicts the following actions.
(a) The target person (the president P) encounters materials and records indicating that the Ainu people currently residing in Hokkaido, Japan, were originally residents of the country R, and were indigenous people of Japan.
(b) The target person (the president P) learns that the Ainu people are being mistreated by the Japanese government.

The predicted action to be taken by the target person in response to the above-mentioned stimuli (input) is "to invade Hokkaido, and annex Hokkaido to the country R to thereby rescue the Ainu people."

### 5. Behavior modification actions made by the behavior modification AI

In response to the behavioral prediction (invasion of Hokkaido) having been output by the behavior prediction AI, the third program 131C (the behavior modification system) formulates behavior modification actions to modify behavior of the target person.

As mentioned earlier, the third program 131C (the behavior modification system) formulates both weakening behavior modification actions and substitutive behavior modification actions.

The weakening behavior modification actions formulated by the behavior modification AI include the following actions, for instance.
(A) Issuing visible signals (for instance, public declarations to the international community, raising the issue at the United Nations, strengthening deployment of the Self-Defense Forces in Hokkaido).
   For instance, the third program 131C (the behavior modification system) proposes these actions to the user of the behavior modification induction system 300, that is, the Japanese government.
(B) Promoting the media to awaken international public opinion.
   For example, the third program 131C (the behavior modification system) urges the user of the behavior modification induction system 300, that is, the Japanese government, to provide necessary information to the media and request the media to publicate relevant articles.
(C) Foretelling economic sanctions (for instance, banning purchase of oil from the country R) through statements or media.

For instance, the third program 131C (the behavior modification system) proposes such actions to the user of the behavior modification induction system 300, that is, the Japanese government.

The substitutive behavior modification actions formulated by the behavior modification AI include the following actions, for instance.

(D) Supporting the country U to strengthen offensive capabilities of the country U being currently in conflict with the country R, to thereby dissuade the country R from invading Hokkaido.

(E) Proposing economic aid beneficial to the country R to create a relaxation of tensions between Japan and the country R.

In either case, the third program 131C (the behavior modification system) formulates such plans and proposes them to the user of the behavior modification induction system 300, that is, the Japanese government.

## Claims

1. A system (300) for designing intervention(s) to introduce a target person (172) to modify his/her actions,
**characterized by**:
a first device (132) for storing therein biometric information about the target person (172), the biometric information including reactions having been made by the target person (172) in response to past external stimulation directed to the target person (172);
a second device (131A) for recreating thought pattern and/or action pattern of the target person (172) through the use of prediction model (210) having been machine-learned using the biometric information as teacher data (200);
a third device (131B) receiving, as input, present external stimulation and/or expected future external simulation directed to the target person (172), and outputting expected reactions of the target person (172) to be made in response to those external stimulation, based on the thought pattern and/or action pattern having been recreated by the second device (131A); and
a fourth device (131C) for designing intervention(s) to introduce the target person (172) to take target action(s) in dependence on the expected reactions of the target person (172) having been output by the third device (131B).

2. The system (300) as set forth in claim 1, wherein the biometric information includes brain pattern information of the target person (172).

3. The system (300) as set forth in claim 1, wherein the fourth device (131C) designs at least one of first intervention and second intervention, the first intervention introducing the target person (172) to weaken his/her emotion arisen by the external stimulation, the second intervention introducing the target person (172) to replace his/her emotion arisen by the external stimulation with other emotion.

4. A portable wireless communication device including the system (300) as set forth in claim 1, 2 or 3.

5. A promotion system for designing and carrying out intervention(s) to introduce a target person (172) to modify his/her actions, including an introduction system (300) and a portable wireless communication device (173) owned by the target person (172),
the introduction system (300) including:
a first device (132) for storing therein biometric information about the target person (172), the biometric information including reactions having been made by the target person (172) in response to past external stimulation directed to the target person (172);
a second device (131A) for recreating thought pattern and/or action pattern of the target person (172) through the use of prediction model (210) having been machine-learned using the biometric information as teacher data (200);
a third device (131B) receiving, as input, present external stimulation and/or expected future external simulation directed to the target person (172), and outputting expected reactions of the target person (172) to be made in response to those external stimulation, based on the thought pattern and/or action pattern having been recreated by the second device (131A); and
a fourth device (131C) for designing a policy of intervention(s) to introduce the target person (172) to take target action(s) in dependence on the expected reactions of the target person (172) having been output by the third device (131B),
the portable wireless communication device (173) storing therein an application (174) for designing intervention(s) based on the policy,
the introduction system (300) transmitting the policy to the portable wireless communication device (173) of the target person (172),
the application (174), on receipt of the policy, designing action of intervention(s) customized for the target person (172) in accordance with the policy.

6. The promotion system (300) as set forth in claim 5, wherein the biometric information includes brain pattern information of the target person (172).

7. The promotion system (300) as set forth in claim 5, wherein the fourth device (131C) designs at least one of first intervention and second intervention, the first intervention introducing the target person (172) to weaken his/her emotion arisen by the external stimulation, the second intervention introducing the target person (172) to replace his/her emotion arisen by the external stimulation with other emotion.

8. A method of designing intervention(s) to introduce a target person (172) to modify his/her actions,
**Characterized by**:
storing (S110) biometric information about the target person (172), the biometric information including reactions having been made by the target person (172) in response to past external stimulation directed to the target person (172);
recreating (S130) thought pattern and/or action pattern of the target person (172) through the use of prediction model (210) having been machine-learned using the biometric information as teacher data (200);
receiving (S140), as input, present external stimulation and/or expected future external simulation directed to the target person (172) to thereby output expected reactions of the target person (172) to be made in response to those external stimulation, based on the thought pattern and/or action pattern having been recreated in the recreating step (S130); and
designing (S160) intervention(s) to introduce the target person (172) to take target action(s) in dependence on the expected reactions of the target person (172) having been output in the receiving step (S140).

9. The method as set forth in claim 8, further including:
designing (S160) a policy of intervention(s) in place of the intervention(s); and
designing (S170) action of intervention(s) customized for the target person (172) in accordance with the policy having been designed in the previous step (S160), the action of intervention(s) being designed by an application (174) stored in a portable wireless communication device (173) owned by the target person (172).

10. The method as set forth in claim 8 or 9, wherein at least one of first intervention and second intervention is designed in the designing step (S160), the first intervention introducing the target person (172) to weaken his/her emotion arisen by the external stimulation, the second intervention introducing the target person (172) to replace his/her emotion arisen by the external stimulation with other emotion.

11. A computer program adapted to perform the steps of the method as set forth in claim 8 or 9, when the program is run on a computer.
